(19) Europäisches Patentamt
European Patent Office
Office européen des brevets

(11) Veröffentlichungsnummer : **0 107 208**
**B1**

(12) # EUROPÄISCHE PATENTSCHRIFT

(45) Veröffentlichungstag der Patentschrift : **22.01.86**

(51) Int. Cl.⁴ : **C 07 C 93/06**, A 61 K 31/135

(21) Anmeldenummer : **83110701.6**

(22) Anmeldetag : **26.10.83**

(54) **Neue 1,1,2-Triphenyl-but-1-en-Derivate, sowie Verfahren zu ihrer Herstellung und ihre Verwendung als Arzneimittel.**

(30) Priorität : **26.10.82 DE 3239610**

(43) Veröffentlichungstag der Anmeldung : **02.05.84 Patentblatt 84/18**

(45) Bekanntmachung des Hinweises auf die Patenterteilung : **22.01.86 Patentblatt 86/04**

(84) Benannte Vertragsstaaten : **AT BE CH DE FR GB IT LI LU NL SE**

(56) Entgegenhaltungen :
**EP-A- 0 002 097**
**EP-A- 0 054 168**
**DD-A- 147 234**
**FR-A- 2 401 132**
**GB-A- 1 099 093**
**Die Akte enthält technische Angaben, die nach dem Eingang der Anmeldung eingereicht wurden und die nicht in dieser Patentschrift enthalten sind.**

(73) Patentinhaber : **Klinge Pharma GmbH**
**Berg-am-Laim-Strasse 129**
**D-8000 München 80 (DE)**

(72) Erfinder : **Schickaneder, Helmut**
**Moosäcker 25**
**D-8501 Eckental-Eckenhaid (DE)**
Erfinder : **Löser, Roland**
**Fichtenweg 12**
**D-8133 Feldafing (DE)**
Erfinder : **Grill, Helmut**
**Zugspitzstrasse 148**
**D-8011 Vaterstetten (DE)**

(74) Vertreter : **Wuesthoff, Franz, Dr.-Ing. et al**
**Patentanwälte Wuesthoff -v. Pechmann-Behrens-Goetz Schweigerstrasse 2**
**D-8000 München 90 (DE)**

**EP 0 107 208 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf neue 1,1,2-Triphenyl-but-1-en-Derivate, welche wertvolle, therapeutisch anwendbare Eigenschaften besitzen.

Aus der britischen Patentschrift 1,013,907 geht hervor, daß 1,1,2-Triphenyl-alken-Derivate antiestrogene Eigenschaften besitzen können und damit zur Behandlung von von hormonabhängigen Tumoren in Frage kommen. Eines davon, das (Z)-1-[4'-(2-Dimethylaminoethoxy) phenyl]-1,2-diphenyl-but-1-en (Tamoxifen, INR rec.) hat sich bereits in der Therapie des hormonabhängigen Mammatumors bewährt.

In der DE-OS 2 807 599 ist festgestellt worden, daß ein Metabolit des Tamoxifens, das (Z)-1-[4'-(2-Dimethylaminoethoxy) phenyl]-(4'-(2-Dimethylaminoethoxy) phenyl-but-1-en, ähnlich starke antiestrogene Aktivität aufweist. Dies trifft auch auf eine Reihe von (Z)-1-[4'-hydroxyphenyl)-2-phenyl-but-1-(4'-hydroxyphenyl)-2-phenyl-but-1-en-Derivaten zu, wie sie in der Europ. Anmeldung 0 002 097 beschrieben sind. Aus der DE-OS 3 046 719 geht hervor, daß auch (E)-1-[4'-(2-Alkylaminoethoxy) phenyl]-1-(3'-hydroxyphenyl)-2-phenyl-but-1-ene ausgeprägte antiestrogene Eigenschaften besitzen.

In einem hochspezifischen Testverfahren wurde nun festgestellt, daß eine Reihe neuer 1,1,2-Triphenyl-but-1-en-Derivate in ihrer antiestrogenen Wirkung dem Tamoxifen deutlich überlegen sind. Wie noch gezeigt wird, hemmen Verbindungen der allgemeinen Formel (1) das Wachstum von Mammatumorzellen viel stärker als Tamoxifen. Dies steht im Einklang mit ihrer stärkeren Bindungsaffinität zum Estrogenrezeptor.

Gegenstand der Erfindung sind 1,1,2-Triphenyl-but-1-en-Derivate der allgemeinen Formel (1), deren Konfiguration der E-Form entspricht,

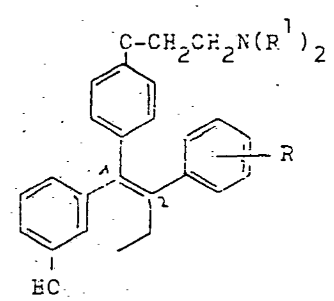

E-Form

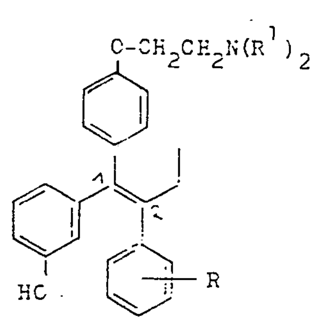

Z-Form

(1)

worin R in Position 3' oder 4' eine Methylgruppe, Methoxygruppe, Hydroxygruppe oder ein Halogenatom und $R^1$ eine niedere Alkylgruppe darstellt. Die niedere Alkylgruppe kann 1 bis 4, vorzugsweise 1 bis 3, C-Atome enthalten.

In dieser Beschreibung beziehen sich die Bezeichnungen E- bzw. Z-Form (E = « entgegen », Z = « zusammen ») auf die Stellung der 3'-Hydroxyphenylgruppe (Priorität 1 am C-Atom 1) zur Stellung der substituierten Phenylgruppe (Priorität 1 am C-Atom 2) an der Doppelbindung in der Butenkette [Nomenklaturregel : R. T. Morrison, R. N. Boyd, Lehrbuch der Organischen Chemie, Verlag Chemie, S. 167 (1974)].

Die E- und Z-Formen unterscheiden sich deutlich in ihren Protonenresonanzsignalen der Dialkylaminogruppe und der O-$CH_2$-Gruppe in der —O—$CH_2CH_2N(CH_3)_2$-Seitenkette. Die Signale der E-Form sind bei den beanspruchten Verbindungen gegenüber der Z-Form hochfeldverschoben. [D. J. Collins, J. J. Hobbs und C. W. Emmers J. Med. Chem. *14*, 952 (1971)].

Die Erfindung betrifft ferner ein Verfahren zur Herstellung von Verbindungen der allgemeinen Formel (1), das dadurch gekennzeichnet ist, daß man Carbinole der allgemeinen Formel (2)

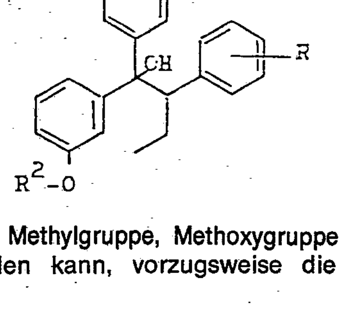

(2)

in der R in Position 3' oder 4' eine Methylgruppe, Methoxygruppe eine Schutzgruppe, die leicht in eine Hydroxygruppe umgewandelt werden kann, vorzugsweise die Acetoxy- bzw. Tetrahydropyranyloxygruppe oder ein Halogenatom und $R^1$ eine niedere Alkylgruppe sein kann und $R^2$ eine leicht hydrolysierbare Schutzgruppe darstellt, in an sich bekannter Weise durch Einwirken von Mineralsäure, unter Abspaltung der Schutzgruppen, dehydratisiert, aus dem anfallenden Isomerenpaar die E-Form isoliert und gegebenenfalls in ein therapeutisch verträgliches Salz überführt.

Als leicht hydrolysierbare Schutzgruppe wird der Tetrahydropyranylrest bevorzugt. Die Abspaltung der Schutzgruppe und die Dehydratisierung gelingt mit Mineralsäure in alkoholischem Milieu, bevorzugt in salzsaurer ethanolischer Lösung. Die Trennung des Isomerenpaares kann sowohl durch Kristallisation als auch mittels chromatographischer Methoden durchgeführt werden. Je nach Löslichkeit werden zur Trennung die freien Basen oder Säure-Additions-Salze eingesetzt.

Die zur Synthese der erfindungsgemäßen Verbindungen notwendigen Ausgangsmaterialien, lassen sich beispielsweise nach folgenden Verfahren herstellen:

Durch Umsetzung von Kaliumphenolat mit 2-Chlorethyl-N,N-dialkylammoniumchlorid wird bei erhöhter Temperatur in ethanolischer Kalilauge und anschließendem Überführen des Reaktionsproduktes in das Hydrochlorid die Verbindung (3) erhalten,

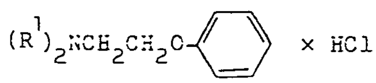
(3)

worin $R^1$ die bei Formel (2) angegebene Bedeutung hat.

In einer Friedel-Crafts-Reaktion von (3) mit substituierten Phenylacetylchloriden der allgemeinen Formel (4)

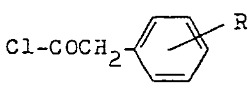
(4)

in der R in Position 3' oder 4' eine Methylgruppe, Methoxygruppe eine Schutzgruppe, die leicht in eine Hydroxygruppe umgewandelt werden kann, vorzugsweise die Acetoxy- oder Tetrahydropyranyloxygruppe oder ein Halogenatom sein kann werden Ethanone der allgemeinen Formel (5) erhalten,

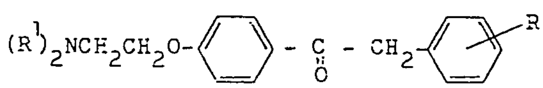
(5)

in der R die in Formel (4) angegebene Bedeutung besitzt.

Verbindungen der allgemeinen Formel (5) setzen sich in Dimethylformamid in Gegenwart von Natriumhydrid mit Ethylbromid zu substituierten Butanonen der allgemeinen Formel (6) um,

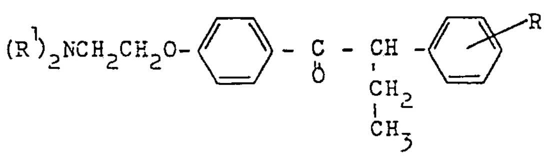
(6)

in der R die in Formel (4) angegebene Bedeutung besitzt.

Verbindungen der allgemeinen Formel (6) reagieren in wasserfreiem Tetrahydrofuran mit 3'-(2-Tetrahydropyranyloxy)-phenyl-magnesiumbromid zu den diastereomeren Carbinolen der allgemeinen Formel (7)

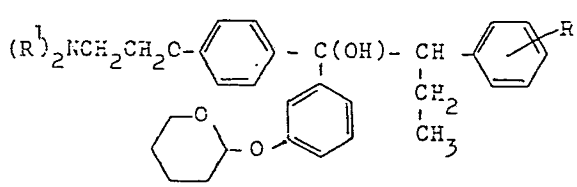
(7)

in der R die in Formel (4) angegebene Bedeutung besitzt und $R^1$ die in Formel (2) angegebene Bedeutung hat.

Verbindungen der allgemeinen Formel (7) spalten in Gegenwart von Mineralsäure bereits bei Raumtemperatur in alkoholischer Lösung den Tetrahydropyranylrest ab und lassen sich in der Hitze unter Dehydratisierung, gegebenenfalls unter Abspaltung einer weiteren Schutzgruppe in ein Isomerenpaar

überführen, aus dem sowohl in seiner Salz- als auch in seiner Basenform das E-Isomere isoliert werden kann, sodaß Verbindungen der allgemeinen Formel (1) nach Anspruch 1 erhalten werden.

Folgende Verbindungen belegen die Ansprüche :

Tabelle 1

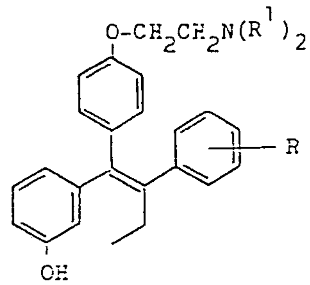

(1)

| Verbindung No. | $R^1$ | R | Fp $[^{\circ}C]^{1)}$ | |
|---|---|---|---|---|
| 1 | $-CH_3$ | $3'-CH_3$ | 169 | (a) |
| 2 | $-CH_3$ | $4'-CH_3$ | 166 | (a) |
| 3 | $-CH_3$ | $4'-OCH_3$ | 133 bis 134 | (a) |
| 4 | $-CH_3$ | $3'-OH$ | 186 | (b) |
| 5 | $-CH_3$ | $4'-Cl$ | 161 bis 162 | (a) |
| 6 | $-CH_3$ | $4'-Br$ | 169 bis 170 | (a) |
| 7 | $-C_2H_5$ | $4'-OCH_3$ | 128 bis 130 | (c) |

1) Kristalle aus : (a) Aceton, (b) Ether/Petrolether, (c) Acetonitril

Die Überlegenheit der beanspruchten Verbindungen läßt sich in hochspezifischen Testverfahren eindeutig belegen.

a) Bindungsaffinität zum Estradiolrezeptor

Die Messung der Bindungsaffinität zum Estradiolrezeptor erfolgte nach der Methode von N. Devleeschouwer, G. Leclercq, A. Danguy und J. C. Heuson [Europ. J. Cancer, *14,* 721-723 (1970)].

Das Uteruscytosol von weiblichen, präpubertären, weißen, 2 kg schweren Kaninchen (Neuseeländer) wurde 18 Stdn. bei 4 °C mit $2.5 \times 10^{-9}$ M [$^3$H]-Estradiol sowie jeweils unter Zusatz von unmarkiertem Estradiol (Kontrolle) bzw. Testsubstanz verschiedener Konzentration inkubiert. Die Bindungsaffinität zum Estradiolrezeptor wird ausgedrückt durch die den Uteruscytosol zugesetzte Konzentration an unmarkiertem Estradiol (Kontrolle) bzw. Testsubstanz, welche eine 5C-proz. Verdrängung des am Estradiolrezeptor gebundenen [$^3$H]-Estradiol bewirkt.

Tabelle 2

Bindungsaffinität der Testsubstanzen

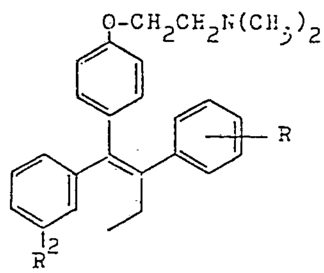

| Verbindung | $R^2$ | R | $ED_{50\%}$ [M]$^{x)}$ |
|---|---|---|---|
| Estradiol (Kontrolle) | | | $1.8 \times 10^{-9}$ |
| Tamoxifen | H | H | $2.5 \times 10^{-7}$ |
| 1 | OH | 3'-$CH_3$ | $3.5 \times 10^{-8}$ |
| 2 | OH | 4'-$CH_3$ | $7.3 \times 10^{-8}$ |
| 3 | OH | 4'-$OCH_3$ | $2.8 \times 10^{-8}$ |
| 5 | OH | 4'-Cl | $8.5 \times 10^{-8}$ |

x) Molare Konzentration der Substanz, die 50 % [$^3$H]-Estradiol vom Estradiol-Rezeptor verdrängt.

Die Affinität der beanspruchten Verbindungen ist zum Estradiol-Rezeptor 3 bis 9 mal höher als bei Tamoxifen.

b) Hemmung der RNS-Synthese in isolierten Human-Mammatumor-Zellen

Seit einigen Jahren ist es gelungen Estrogenrezeptor positive [ER +] Mammatumor-Zellen aus Patienten zu isolieren und *in vitro* kontinuierlich zu züchten. Diese sog. Zell-Linien enthalten nahezu alle in vivo beobachteten morphologischen Charakteristika und Stoffwechselleistungen. Sie stellen somit ein ideales Modell zur Testung von Antiestrogenen dar, da direkte Aussagen über den Einfluß auf den *in vivo* zu therapierenden Zelltyp gemacht werden können.

Zur Prüfung der antiestrogenen Eigenschaften der beanspruchten Verbindungen wurden Estrogenrezeptor positive Human-Mammatumor-Zellen der Zell-Linie ZR-75-1 eingesetzt, wie sie von Engel, L. W. et al beschrieben wurden [Linda W. Engel et al. Cancer Research *38*, 3552-3364 (1978)]. Der Einfluß der beanspruchten Verbindungen auf das Wachstum dieser Zellen wurde über den Einbau von markiertem Uridin bzw. Thymidin nach der Methode von Lippman, M. et al kontrolliert. [Marc Lippman et al, Cancer Research *36*, 4595-4601 (1976)].

Es wurden ZR-75-I-Zellen 48 Stunden mit Tamoxifen oder mit einer der beanspruchten Verbindungen in Konzentrationen von $4 \times 10^{-6}$ [M] bis $1 \times 10^{-7}$ [M] inkubiert, anschließend mit markiertem Uridin bzw. Thymidin versetzt und nach einer Stunde die Einbaurate des markierten Materials in den Zellen bestimmt. Tabelle 3 zeigt die prozentuale Hemmung der zellulären RNS-Synthese durch die beanspruchten Verbindungen im Vergleich zu Tamoxifen.

Zur Nachahmung der Verhältnisse *in vivo* wurde die Potenz der antiestrogenen Wirkung der beanspruchten Verbindungen in Gegenwart eines Estrogens geprüft.

Es wurden ZR-75-I-Zellen gleichzeitig mit einer der beanspruchten Verbindungen in einer Konzentration von $1 \times 10^6$ [M] und mit 17-β-Estradiol ($1 \times 10^{-8}$ [M]) versetzt, nach 48-stündiger Inkubation markiertes Uridin bzw. Thymidin hinzugefügt und nach einer Stunde die Einbaurate des markierten Materials in den Zellen bestimmt. [Tab. 4]

(Siehe Tabelle 3 Seite 6 f.)

Tabelle 3

Prozentuale Hemmung der RNS-Synthese in ZR-75-I-Zellen im Vergleich mit Taxoxifen

| | Substanzkonzentrationen | | |
|---|---|---|---|
| | $4 \times 10^{-6}$ [M] | $1 \times 10^{-6}$ [M] | $1 \times 10^{-7}$ [M] |
| Verbindung No. | Prozentuale Hemmung | | |
| 1 | 100 | 74 | 47 |
| Tamoxifen | 100 | 44 | 29 |
| 2 | 100 | 62 | 42 |
| Tamoxifen | 100 | 39 | 16 |
| 3 | 100 | 68 | 36 |
| Tamoxifen | 99 | 43 | 13 |
| 4 | 100 | 73 | 33 |
| Tamoxifen | 100 | 44 | 29 |
| 5 | 100 | 61 | 41 |
| Tamoxifen | 100 | 39 | 16 |
| 6 | 100 | 70 | 47 |
| Tamoxifen | 100 | 59 | 43 |
| 7 | 91 | 37 | 4 |
| Taxoxifen | 93 | 46 | 2 |

Wie aus Tabelle 3 hervorgeht, hemmen die beanspruchten Verbindungen die RNS-Synthese in ZR-75-I-Zellen ab einer Verdünnung von $1 \times 10^{-6}$ [M] viel stärker als Tamoxifen, das jeweils bei dem betreffenden Versuch als Vergleichssubstanz herangezogen worden ist.

Tabelle 4

Prozentuale Hemmung der RNS-Synthese in ZR-75-I-Zellen

| | Substanzkonzentrationen | |
|---|---|---|
| | Verbindung $1 \times 10^{-6}$ [M] | Verbindung + 17-β-Estradiol $1 \times 10^{-6}$ [M] $1 \times 10^{-8}$ [M] |
| Verbindung No. | Prozentuale Hemmung | |
| 1 | 74 | 74 |
| 2 | 62 | 58 |

(Fortsetzung)

| | Substanzkonzentrationen | |
|---|---|---|
| | Verbindung $1 \times 10^{-6}$ [M] | Verbindung + 17-β-Estradiol $1 \times 10^{-6}$ [M] $1 \times 10^{-8}$ [M] |
| 3 | 68 | 65 |
| 4 | 73 | 79 |
| 6 | 70 | 74 |
| 7 | 37 | 41 |

Wie aus Tab. 4 hervorgeht hemmen die beanspruchten Verbindungen auch in Gegenwart einer relativ hohen Konzentration an 17-β-Estradiol ungehindert die RNS-Synthese in ZR-75-I-Zellen.

Die erfindungsgemäßen Verbindungen stellen somit eine wertvolle Bereicherung des Arzneimittelschatzes dar und können zur Behandlung des malignen Mammatumors eingesetzt werden.

Die Erfindung betrifft außerdem Arzneimittel, die eine Verbindung der allgemeinen Formel (1) als Wirkstoff neben den üblichen pharmazeutischen Träger- und Hilfsstoffen enthalten.

Die beanspruchten Verbindungen werden bevorzugt oral verabreicht. Gewöhnlich beträgt die orale Tagesdosis 0,1 bis 3,0 mg/kg Körpergewicht, vorzugsweise 0,3 bis 2,0 mg/kg Körpergewicht. Trotzdem kann es gegebenenfalls erforderlich sein, von den genannten Dosen abzuweichen, und zwar in Abhängigkeit des individuellen Verhaltens gegenüber dem Medikament bzw. der Art seiner Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen. Die Wirkstoffe können in üblicher Form zur oralen Verabreichung konfektioniert werden, z. B. in Kapseln, als Tabletten oder als Dragees. Die Freisetzung der beanspruchten Verbindungen kann je nach pharmazeutischer Bearbeitung beschleunigt oder verzögert werden.

Durch Vermischen mit festen, pulverförmigen Trägerstoffen, wie mikronisierte Cellulose, Kartoffel- oder Maisstärke, mit Zusätzen wie Natriumcitrat, Calcium-carbonat und Bindemitteln wie Polyvinylpyrrolidon, Gelatine oder Cellulosederivaten, gegebenenfalls unter Zusatz von Gleitmitteln wie Magnesiumstearat, Natriumlaurylsulfat oder Polyethylenglykolen, können sie zu Tabletten oder zu Drageekernen verarbeitet werden. Selbstverständlich können bei den oralen Verabreichungsformen Geschmackskorrigenzien zugesetzt werden.

Als weitere Verabreichungsformen eignen sich Steckkapseln, z. B. aus Hartgelatine, sowie geschlossene Weichgelatinekapseln mit einem Weichmacher wie z. B. Glycerin. Die Steckkapseln enthalten den Wirkstoff vorzugsweise als Granulat, z. B. in Mischung mit Füllstoffen wie Lactose, Saccharose, Mannit, Stärken wie z. B. Kartoffelstärke oder Amylopektin, Cellulosederivaten oder hochdispersen Kieselsäuren. In Weichgelatinekapseln ist der Wirkstoff vorzugsweise in geeigneten Flüssigkeiten, z. B. in Pflanzenölen oder in flüssigen Polyethylenglykolen gelöst oder suspendiert.

Die Herstellung der Vorstufen erfolgt nach bekannten Verfahren, wie sie teilweise in der DE-PS 3 046 719 beschrieben sind.

Herstellung der Vorstufen

a) N,N-Dimethyl-2-phenoxyethylammoniumchlorid

117 g (1.8 Mol) Kaliumhydroxid und 94.1 g (1.0 Mol) Phenol werden in 500 mL Ethanol gelöst, mit einer Suspension von 144 g (1.0 Mol) 2-Chlorethyl-N,N-dimethylammoniumchlorid in 500 mL Ethanol versetzt und unter kräftigem Rühren 1 Stde. unter Rückfluß erhitzt. Nach dem Abkühlen saugt man vom abgeschiedenen Kaliumchlorid ab, wäscht mit Ethanol nach und engt das Filtrat i. Vak. zur Trockne ein. Den Rückstand nimmt man mit Ether auf, wäscht mehrfach mit 10proz. Natronlauge, dann mit Wasser und trocknet über Natriumsulfat. Durch Einleiten von trockenem Chlorwasserstoff wird N,N-Dimethyl-2-phenoxyethylammoniumchlorid erhalten, das aus Isopropanol umgelöst werden kann. Farblose Kristalle vom Schmp. 103 °C. Ausbeute 104.7 g.

b) 1-[4'-(2-Dimethylaminoethoxy)phenyl]-2-(subst. phenyl)-ethan-1-one

20.1 g (0.1 Mol) N,N-Dimethyl-2-phenoxyethylammoniumchlorid und 0.12 Mol eines entsprechend substituierten Phenylacetylchlorids in 1 L Dichlormethan werden bei Raumtemperatur unter kräftigem Rühren portionsweise mit 24.7 g (0.18 Mol) wasserfreiem Aluminiumchlorid versetzt, anschließend langsam erwärmt und 2 Stdn. unter Rückfluß erhitzt. Nach dem Abkühlen gießt man auf Eis, fügt 100 ml konz. Salzsäure hinzu, trennt ab und schüttelt die organische Phase noch zweimal mit 10proz. Salzsäure aus. Die vereinigten wäßrigen Lösungen werden alkalisiert und dreimal mit je 200 mL Ethylacetat extrahiert. Nach dem Waschen mit Wasser und Trocknen über Natriumsulfat destilliert man das Lösungsmittel i. Vak. ab und kristallisiert das anfallende 1-[4'-(2-Dimethylaminoethoxy)phenyl]-2-(subst. phenyl)-ethan-1-on aus Petrolether um.

c) 1-[4'-(2-Dimethylaminoethoxy) phenyl]-2-(subst. phenyl)-butan-1-one

Die Darstellung erfolgt durch Umsetzung eines entsprechenden 1-[4'-(2-Dimethylaminoethoxy) phenyl]-2-(subst. phenyl)-ethan-1-on mit Ethylbromid und Natriumhydrid in wasserfreiem Dimethylformamid, wie in DE-PS 3 046 719 (Beispiel 1b) detailliert beschrieben.

d) 1-[4'-(2-Dimethylaminoethoxy) phenyl]-2-(subst. phenyl)-1-[3'-(2-tetrahydropyranyloxy) phenyl]-butan-1-ol (Diastereomere)

Die Darstellung erfolgt durch Umsetzung der 1-[4'-(2-Dimethylaminoethoxy) phenyl]-2-(subst. phenyl)butan-1-one mit 3'-(2-Tetrahydropyranyloxy) phenylmagnesiumbromid in wasserfreiem Tetrahydrofuran, wie in DE-PS 3 046 719 (Beispiel 1 e) detailliert beschrieben.

Im folgenden wird das beanspruchte Verfahren anhand von Ausführungsbeispielen näher beschrieben.

Beispiel 1

Erfindungsgemäße Herstellung von (E)-1-[4'-(2-Dimethylaminoethoxy) phenyl]-1-(3'-hydroxyphenyl)-2-(4'-methoxyphenyl)-but-1-en (Verbindung No. 3)

51.9 g (0.1 Mol) des Diastereomeren-Gemisches von 1-[4'-(2-Dimethylaminoethoxy) phenyl]-2-(4'-methoxyphenyl)-1-[3'-(2-tetrahydropyranyloxy) phenyl]-butan-1-ol in 1.5 L Ethanol werden mit 60 mL konz. Salzsäure versetzt und 2 Stdn. unter Rückfluß erhitzt. Anschließend wird das Lösungsmittel i. Vak. entfernt, der Rückstand in 200 mL verd. Ammoniaklösung suspendiert und zweimal mit je 250 mL Ethylacetat ausgeschüttelt. Die organische Phase wird mit Wasser neutral gewaschen und nach dem Trocknen über Natriumsulfat das Lösungsmittel i. Vak. entfernt. Der Rückstand wird aus Aceton kristallisiert. Farblose Kristalle vom Schmp. 133 bis 134 °C ; $R_f$ 0.35 [$CHCl_3/CH_3OH$ (7/3)] ; Ausbeute 8.34 g (20 %).

| | | | |
|---|---|---|---|
| $C_{27}H_{31}NO_3$ | (417.5) | | |
| Ber. : | C 77.66 | H 7.48 | N 3.36 |
| Gef. : | C 77.45 | H 7.52 | N 3.31 |

| | | | |
|---|---|---|---|
| Mol.-Masse 417 | (massenspektrometrisch bestimmt) | | |
| IR-Spektrum (Kbr) | : ν (O—H) 3 650 bis 2 600 $cm^{-1}$ | | |
| $^1H$-NMR-Spektrum *) | | | |
| ($CDCl_3$) | : 0.90 | t | (3) $C\underline{H}_3$ [J = 7.0] |
| | 2.33 | s | (6) $(C\underline{H}_3)_2N$ |
| | 2.43 bis 2.87 | m | (4) $C\underline{H}_2$ und $C\underline{H}_2N$ |
| | 3.63 bis 4.10 | t | (5) $C\underline{H}_2O$ und $C\underline{H}_3O$ |
| | und 3.80 | s | |
| | 5.23 breit | s | (1) O$\underline{H}$ [austauschbar mit $D_2O$] |
| | 6.33 bis 7.47 | m | (12) Aromaten-$\underline{H}$ |

*) aufgenommen bei 60 MHz ; die chemischen Verschiebungen sind in ppm gegen TMS (= 0,0) angegeben, die relativen Intensitäten sind in Klammern beigefügt. s = Singulett ; d = Dublett ; t = Triplett ; m = Multiplett ; J = Kopplungskonstante in Hz. δ = 0.0.

## Beispiel 2

Erfindungsgemäße Herstellung von (E)-1-[4'-(2-Dimethylaminoethoxy) phenyl]-1,2-bis-(3'-hydroxyphenyl)-but-1-en (Verbindung No. 4)

58.9 g (0.1 Mol) des Diastereomeren-Gemisches von 1-[4'-(2-Dimethylaminoethoxy) phenyl]-1,2-bis-[3'-(2-tetrahydropyranyloxy)-phenyl]-butan-1-ol in 1 L 95-proz. Ethanol werden mit 30 mL konz. Salzsäure versetzt, dann 2 Stdn. unter Rückfluß erhitzt und wie in Beispiel 1 beschrieben, aufgearbeitet. Farblose, lichtempfindliche Kristalle vom Schmp. 186 °C [Ether/Petrolether (1/1)] ; $R_f$ 0.20 [$CHCl_3/CH_3OH$ (7/3)] ; Ausbeute 24.2 g (60 %).

$C_{26}H_{29}NO_3$ (403.5)
Ber. : C 77.39 H 7.24 N 3.47
Gef. : C 77.53 H 7.39 N 3.44

| | | | | |
|---|---|---|---|---|
| Mol.-Masse 403 | (massenspektrometrisch bestimmt) | | | |
| IR-Spektrum (KBr) | : $\nu$ (OH) 3 600 bis 2 300 $cm^{-1}$ | | | |
| $^1H$-NMR-Spektrum ($d_6$-DMSO) | : 0.83 | t | (3) | $C\underline{H}_3$ [J = 7.0] |
| | 2.17 | s | (6) | $(C\underline{H}_3)_2N$ |
| | 2.33 bis 2.73 | m | (4) | $C\underline{H}_2$ und $C\underline{H}_2N$ |
| | 3.90 | t | (2) | $C\underline{H}_2O$ [J = 6.0] |
| | 6.40 bis 7.43 | m | (12) | Aromaten-$\underline{H}$ |
| | 9.33 breit | s | (2) | $O\underline{H}$ [austauschbar mit $D_2O$] |

## Beispiel 3

Erfindungsgemäße Herstellung von (E)-2-(4'-Bromphenyl)-1-[4'-(2-dimethylaminoethoxy) phenyl]-1-(3'-hydroxyphenyl)-but-1-en (Verbindung No. 6)

56.8 g (0.1 Mol) des Diastereomeren-Gemisches von 2-(4'-Bromphenyl)-1-[4'-(2-dimethylaminoethoxy) phenyl]-1-[3'-(2-tetrahydropyranyloxy)-phenyl]-butan-1-ol in 500 mL Ethanol werden mit 25 mL konz. Salzsäure versetzt, dann 2 Stdn. unter Rückfluß erhitzt und wie in Beispiel 1 beschrieben aufgearbeitet. Farblose Kristalle vom Schmp. 169 bis 170 °C [Aceton] ; $R_f$ 0.30 [$CHCl_3/CH_3OH$ (7/3)] ;
Ausbeute 30.3 g (65 %).

$C_{26}H_{28}BrNO_2$ (466.4)
Ber. : C 66.95 H 6.05 N 3.00
Gef. : C 66.71 H 5.88 N 3.02

| | | | | |
|---|---|---|---|---|
| Mol.-Masse 465*) | (massenspektrometrisch bestimmt) | | | |
| IR-Spektrum (KBr) | : $\nu$ (C—H) 3 600 bis 2 400 $cm^{-1}$ | | | |
| $^1H$-NMR-Spektrum ($d_6$-DMSO) | : 0.83 | t | (3) | $C\underline{H}_3$ [J = 7.0] |
| | 2.23 | s | (6) | $(C\underline{H}_3)_2N$ |
| | 2.30 bis 2.73 | m | (4) | $C\underline{H}_2$ und $C\underline{H}_2N$ |
| | 3.93 | t | (2) | $C\underline{H}_2O$ [J = 6.0] |
| | 6.50 bis 7.60 | m | (12) | Aromaten-$\underline{H}$ |
| | 9.43 | s | (1) | $O\underline{H}$ [austauschbar mit $D_2O$] |

*) Mol.-Masse mit dem Bromisotop 79

## Beispiel 4

(E)-1-[4'-(2-Dimethylaminoethoxy) phenyl]-1-(3'-hydroxyphenyl)-2-(3'-methylphenyl)-but-1-en-hydrochlorid enthaltendes Arzneimittel

21.82 g pulverisiertes (E)-1-[4'-(2-Dimethylaminoethoxy) phenyl]-1-(3'-hydroxyphenyl)-2-(3'-methylphenyl)-but-1-en-hydrochlorid werden mit 40 g Lactose und 140 g Stärke vermischt, anschließend mit 33 g Talkum und 13 g Calciumstearat versetzt und nach sorgfältiger Durchmischung in zweitausend Hartgelatinekapseln geeigneter Größe abgefüllt, sodaß jede Kapsel 10 mg Wirkstoff (berechnet als freie Base) enthält.

Beispiel 5

(E)-1-[4'-(2-Dimethylaminoethoxy) phenyl]-1-(3'-hydroxyphenyl)-2-(4'-methoxyphenyl)-but-1-en enthaltendes Arzneimittel

20.0 g feingepulvertes (E)-1-[4'-(2-Dimethylaminoethoxy) phenyl]-1-(3'-hydroxyphenyl)-2-(4'-methoxyphenyl)-but-1-en werden nach dem Vermischen mit 111 g Nannit, 15 g Maisstärke und 6 g Alginsäure granuliert und das getrocknete Granulat nach sorgfältigem Vermischen mit 0.75 g Methylcellulose und 1.5 g Magnesiumstearat zu eintausend Tabletten verpreßt, sodaß jede Tablette 20 mg Wirkstoff enthält.

Beispiel 6

Erfindungsgemäße Herstellung von (E)-1-[4'-(2-Diethylaminoethoxy) phenyl]-1-(3'-hydroxyphenyl)-2-(4'methoxyphenyl)-but-1-en

Zur Herstellung des N,N-Diethyl-2-phenoxyethylammoniumchlorids werden 117 g (1.8 Mol) Kaliumhydroxid und 94.1 g (1 Mol) Phenol in 1 L Ethanol mit 172 g (1.0 Mol) 2-Chlorethyl-N,N-diethylammoniumchlorid umgesetzt, wie dies unter a) oben beschrieben ist. Aus Isopropanol werden farblose Kristalle vom Schmp. 136-137 °C in einer Ausbeute von 79 g erhalten. Daraus werden analog den Angaben, wie sie oben in b) bzw. in DE-PS 30 46 719 enthalten sind, das 1-[4'-(2-Diethyl-aminoethoxy) phenyl]-2-(4'-methoxyphenyl)-1-[3'-(2-tetrahydropyranyloxy) phenyl]-butan-1-ol hergestellt.

54,7 g (0.1 Mol) des Diastereomeren-Gemisches von 1-[4'-(2-Diethylaminoethoxy) phenyl]-2-(4'-methoxyphenyl)-1-[3'-(2-tetrahydropyranyloxy)-phenyl]-butan-1-ol in 1 L Ethanol werden mit 30 ml konz. Salzsäure versetzt, dann 2 Stdn. unter Rückfluß erhitzt und wie in Beispiel 1 beschrieben aufgearbeitet. Farblose, lichtempfindliche Kristalle vom Schmp. 128-130 °C (Acetonitril) ; $R_f$ 0,46 [Benzol/Triethylamin (94/6)] ;

Ausbeute 7,6 g (20 %).

$C_{29}H_{35}NO_3$ (445,6)

| | | | | |
|---|---|---|---|---|
| Mol.-Masse 445 | (massenspektrometrisch bestimmt) | | | |
| $^1$H-NMR-Spektrum (CDCl$_3$) | : 0,70 bis 1,23 | m | (9) | $3CH_3$ |
| | 2,23 bis 2,97 | m | (8) | $3CH_2N$ und $C=CCH_2$ |
| | 3,67 bis 4,03 | t | | |
| | und 3,73 | s | (5) | $CH_2O$ und $CH_3O$ |
| | 6,27 bis 7,30 | m | (12) | Aromaten-H |
| | 7,8 | s | (1) | OH [austauschbar mit $D_2O$] |

**Patentansprüche**

1. 1,1,2-Triphenyl-but-1-en-Derivate der allgemeinen Formel (1), deren Konfiguration der E-Form entspricht,

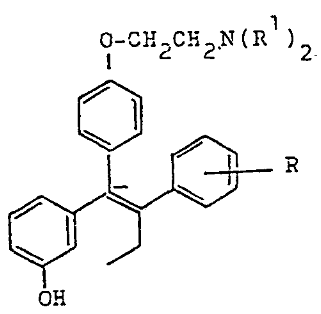

(1)

der R in Position 3' oder 4' eine Methylgruppe, Methoxygruppe, Hydroxygruppe oder ein Halogenatom ıd $R^1$ eine Alkylgruppe mit 1 bis 4 C-Atomen darstellt und deren therapeutisch verträgliche Salze.

2. Verfahren zur Herstellung von 1,1,2-Triphenyl-but-1-en-Derivate nach Anspruch 1, dadurch ekennzeichnet, daß man Carbinole der allgemeinen Formel (2)

(Siehe Schema Seite 11 f.)

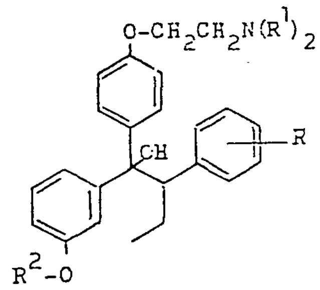

(2)

in der R in Position 3' oder 4' eine Methylgruppe, Methoxygruppe, eine Schutzgruppe, die leicht in eine Hydroxygruppe umgewandelt werden kann, vorzugsweise die Acetoxy- bzw. Tetrahydropyranyloxygruppe oder ein Halogenatom und $R^1$ eine niedere Alkylgruppe sein kann und $R^2$ eine leicht hydrolysierbare Schutzgruppe darstellt, in an sich bekannter Weise durch Einwirken von Mineralsäure, unter Abspaltung der Schutzgruppen, dehydratisiert, aus dem anfallenden Isomerenpaar die E-Form isoliert und gegebenenfalls in ein therapeutisch verträgliches Salz überführt.

3. Arzneimittel enthaltend eine Verbindung gemäß Anspruch 1 als Wirkstoff, sowie übliche Träger- und Hilfsstoffe.

**Claims**

1. 1,1,2-triphenyl-but-1-en-derivatives represented by general formula (1), the configuration of which corresponds to E form

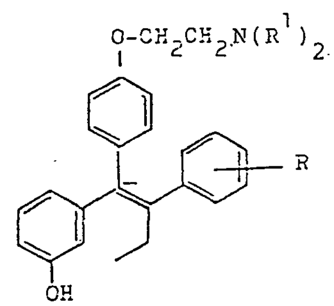

(1)

in which R in position 3' or 4' is a methyl group, methoxy group, hydroxy group or a halogen atom and $R^1$ is an alkyl group with 1 to 4 C atoms, and the therapeutically acceptable salts thereof.

2. A process of producing 1,1,2-triphenyl-but-1-en derivatives as claimed in claim 1, characterized in that carbinoles represented by general formula (2)

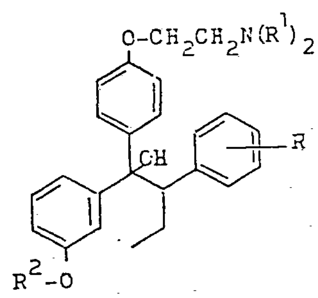

(2)

in which R in position 3' or 4' may be a methyl group, methoxy group, a protecting group which can easily be converted to a hydroxy group, preferrably the acetoxy or tetrahydropyranyloxy group or a halogen atom, and $R^1$ may be a lower alkyl group, and $R^2$ represents an easily hydrolysable protecting group, are dehydrated in a manner known per se by the action of mineral acid while removing the protecting groups, the E form is isolated from the resulting pair of isomers and optionally is converted to a therapeutically acceptable salt.

3. A drug containing a compound according to claim 1 as active ingredient, as well as common carrier and auxiliary substances.

**Revendications**

1. Dérivés de triphényl-1,1,2-butène-1 de formule générale (1) dont la configuration correspond à la forme E

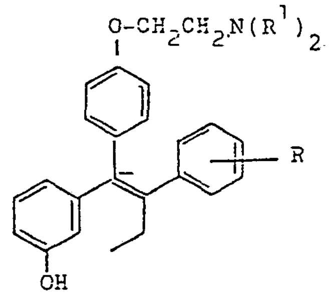

(1)

formule dans laquelle R représente un groupe méthyle, un groupe méthoxy, un groupe hydroxy ou un atome d'halogène en position 3' ou 4' et $R^1$ représente un groupe alkyle avec 1 à 4 atomes de carbone, et leurs sels thérapeutiquement compatibles.

2. Procédé de préparation de dérivés de triphényl-1,1,2-butène-1 selon la revendication 1, caractérisé en ce qu'on déshydrate de façon connue en soi, par action d'un acide minéral avec scission des groupes protecteurs, des carbinols de formule générale (2)

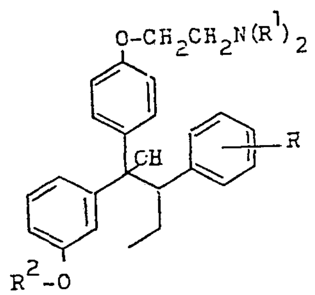

(2)

dans laquelle R peut être un groupe méthyle, un groupe méthoxy, un groupe protecteur pouvant être facilement transformé en un groupe hydroxyle, de préférence le groupe acétoxy ou le groupe tétrahydropyrannyloxy ou un atome d'halogène en position 3' ou 4' et $R^1$ un groupe alkyle inférieur et $R^2$ représente un groupe protecteur facilement hydrolysable, qu'on isole la forme E à partir de la paire d'isomères obtenue et la transforme éventuellement en un sel thérapeutiquement compatible.

3. Médicament contenant, à titre de principe actif, un composé selon la revendication 1, ainsi que les véhicules et adjuvants usuels.